# FASCICULE DE BREVET EUROPEEN

(11) **EP 2 875 789 B1**
(45) Date de publication et mention de la délivrance du brevet: **16.11.2016**
(21) Numéro de dépôt: 14193905.8
(22) Date de dépôt: 19.11.2014
(51) Int. Cl.: A61B 17/80, A61C 8/00

(54) **Outil dentaire à bandes indicatrices de pénétration**
Zahnmedizinisches Instrument mit Streifen, die die Penetration anzeigen
Dental tool with strips for indicating penetration

(30) Priorité: 25.11.2013 FR 1361613
(43) Date de publication de la demande: 27.05.2015
(73) Titulaire: BIOTECH DENTAL, 13300 Salon-de-Provence (FR)
(72) Inventeur: Breysse, Pascal, 69210 ST Germain/L'Arbresle (FR)
(74) Mandataire: Santarelli

(56) Documents cités:
- KR-A- 20120 096 839
- US-A1- 2007 101 827

## Description

L'invention concerne un instrument dentaire, du type foret, fraise ou alésoir, dont la surface de travail comporte un revêtement dur et résistant à l'usure.

Un tel instrument est classiquement de forme allongée en présentant une partie de manoeuvre destinée à être engagée dans un appareil d'entraînement en rotation, manuel ou motorisé, et une partie de travail dont la surface de travail, s'étendant à partir d'une extrémité, assure la fonction de forage ou d'alésage.

Ainsi qu'on le sait une intervention dentaire telle qu'une ostéotomie, implique généralement la formation ou l'agrandissement d'un canal dans l'os de la mâchoire, parfois au moyen d'un instrument entraîné en rotation à grande vitesse, et il est habituellement demandé à un tel instrument dentaire de présenter un faible coefficient de frottement avec l'os de la mâchoire (ce qui est favorisé par l'existence d'une lubrification, parfois au travers de l'instrument), une grande dureté mécanique conférant une bonne résistance à l'usure, une bonne résistance à la corrosion, notamment aux acides de la salive, et une bonne conductivité thermique pour évacuer de manière efficace la chaleur générée lors du forage d'un canal, tout en étant biocompatible.

Pour satisfaire une telle combinaison de besoins, il a été proposé, notamment par le document US - 5 299 937, de recouvrir au moins la surface de travail d'un instrument dentaire par un revêtement de carbone dur du type diamant (on utilise souvent l'acronyme anglo-saxon DLC pour Diamond-Like-Carbon) ; plus récemment, le document EP - 1 128 777 a préconisé de sélectionner un tel revêtement de carbone dur contenant entre 5% at et 35% at d'hydrogène.

En fait, lorsqu'on utilise un instrument dentaire pour ostéotomie, manoeuvré à la main ou actionné au moyen d'un moteur d'entraînement en rotation, il est utile de pouvoir évaluer de manière aussi précise que possible la profondeur à laquelle l'extrémité de l'instrument est parvenue dans l'os. C'est pour satisfaire ce besoin qu'il a été proposé, notamment dans le document EP - 1 128 777 précité, de former sur la surface de travail de l'instrument des repères visuels de profondeur, tels que des bandes annulaires entourant l'instrument dans sa partie de travail réalisées en sorte de former un contraste visuel avec le reste de la surface de cette partie de travail ; en pratique ces bandes de profondeur sont formées en enlevant localement tout ou partie de l'épaisseur du revêtement en carbone dur de type diamant. En pratique ces bandes sont équidistantes ; en outre, il y a des instruments sur lesquels les bandes ont une largeur sensiblement égale à la distance les séparant.

Une telle configuration donne satisfaction dans de nombreux cas mais il a été constaté que, dans certaines orientations, lors de certaines interventions, le contraste visuel entre les bandes de profondeur devient insuffisant pour permettre une évaluation fiable de la profondeur à laquelle l'extrémité de l'instrument utilisé est parvenue.

Le document KR 2012 0096839 A divulgue un instrument dentaire adapté pour réaliser une ostéotomie comportant une tige de montage et, dans le prolongement de celle-ci, une portion de travail comportant des zones de découpe, dont au moins une partie est recouverte par un revêtement en carbure de tungstène enrichi en carbone.

Le document US 2007/101827 A1 divulgue un instrument endodontique ayant un revêtement en carbure de tungstène sur les bords qui leur confère une couleur différente de celle du reste de la partie de travail de l'instrument endodontique.

L'invention a pour objet un instrument dentaire, adapté à des opérations d'ostéotomie, répondant aux diverses contraintes s'appliquant à un tel instrument, notamment celles énoncées ci-dessus, tout en présentant un contraste amélioré par rapport à ce que permet un revêtement en carbone dur du type diamant (DLC).

L'invention propose à cet effet un instrument dentaire adapté à la réalisation d'une ostéotomie comportant une tige de montage et, dans le prolongement de celle-ci, une portion de travail comportant des zones de découpe, dont au moins une partie est recouverte par un revêtement en carbure de tungstène enrichi en carbone à l'exception d'une pluralité de bandes annulaires réparties le long de la longueur de la portion de travail pour indiquer la profondeur de pénétration de l'outil dans une portion de mâchoire.

De manière tout à fait surprenante, il a été constaté que le carbure de tungstène enrichi en carbone permet une meilleure détection des bandes annulaires que le DLC bien que le carbure de tungstène enrichi en carbone soit moins noir que le DLC (le DLC est d'un noir profond alors que le carbure de tungstène enrichi en carbone, désigné dans la suite par l'acronyme CTEC, est d'un gris foncé). En fait, il a initialement été supposé que, pour améliorer le contraste entre les bandes annulaires et le revêtement, il fallait augmenter la différence entre les couleurs entre ces bandes annulaires et le revêtement ; il a toutefois été constaté que ce contraste visuel pouvait être amélioré avec le CTEC, malgré une différence plus faible de couleur, en raison du fait que ce revêtement est mat alors que le DLC peut présenter des reflets dans certaines configurations d'éclairage. En d'autres termes, pour améliorer la détection des bandes annulaires, il pouvait être plus efficace de chercher à obtenir une forte différence d'aspect (d'éclat ou de brillance) plutôt qu'une maximisation de la différence de couleur ; le contraste pouvait être amélioré du point de vue couleur mais aussi et surtout du point de vue brillance.

De manière avantageuse, le revêtement a une épaisseur comprise entre 1 et 5 micromètres, voire entre 1 et 3 micromètres par exemple au moins approximativement égale à 2 micromètres.

Selon une autre caractéristique avantageuse de l'invention, les bandes annulaires sont équidistantes ; en outre, elles ont de préférence une dimension longitudinale égale à leur écartement.

Indépendamment de la dimension longitudinale des bandes, leur écartement est de préférence compris entre 1 et 3 mm, par exemple 2 mm ; par analogie, indépendamment de la dimension longitudinale des écartements entre les bandes (qui peuvent varier d'un emplacement à l'autre), la dimension longitudinale des bandes est avantageusement comprise entre 1 et 3 mm, par exemple égale à 2 mm.

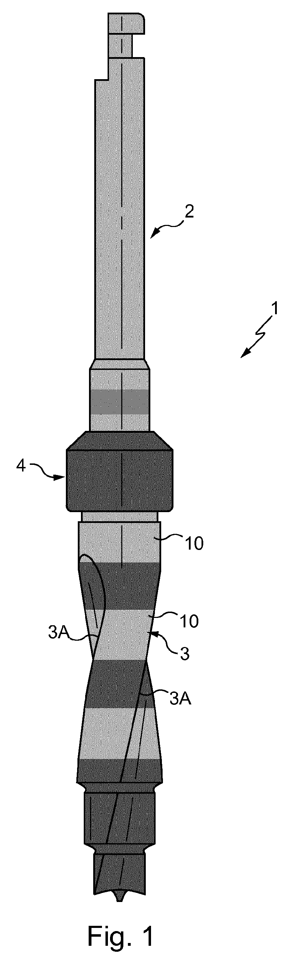

Des objets, caractéristiques et avantages de l'invention ressortent de la description qui suit, donnée à titre d'exemple illustratif non limitatif, en regard du dessin annexé sur lequel la figure 1 unique représente un instrument dentaire conforme à l'invention.

De manière habituelle l'instrument dentaire 1 a une forme allongée, globalement cylindrique avec un diamètre très inférieur à sa longueur.

Dans l'exemple ici considéré, l'instrument dentaire est un foret, mais peut, en variante, être notamment un alésoir, un taraud, notamment, ou tout autre instrument pouvant participer à une intervention d'ostéotomie.

Ce foret comporte, de manière classique une tige de préhension 2 destinée à être engagée dans un embout d'un dispositif d'entraînement à grande vitesse de rotation, et une portion de travail 3 comportant des zones (ici des arêtes) de découpe 3A s'étendant longitudinalement, de manière avantageusement hélicoïdale ; ces zones s'arrêtent ici à une légère distance de l'extrémité de la portion de travail, qui a un diamètre plus faible que la partie dans laquelle sont ménagées ces zones de découpe.

Une surépaisseur annulaire 4 est ici ménagée entre la tige de préhension 2 et la partie de travail 3.

Selon l'invention, au moins une partie de la portion de travail porte un revêtement en carbure de tungstène enrichi en carbone, ou CTEC.

De manière préférée, ce revêtement s'étend depuis l'extrémité de la portion de travail jusqu'à la séparation (auprès de la surépaisseur annulaire 4) entre cette portion de travail et la tige de préhension. En d'autres termes, ce revêtement s'étend sur quasiment toute la longueur de la portion de travail. En variante, il peut ne s'étendre que sur une fraction (en pratique d'au moins 50%, de préférence au moins 75%) de cette longueur.

De manière préférée, ce revêtement recouvre, dans l'exemple représenté, non seulement la surface extérieure de la portion de travail mais aussi l'intérieur des zones situées en retrait vers l'axe par rapport à la forme cylindrique qu'a localement l'outil, c'est à dire dans les zones longeant les arêtes de découpe. En variante, tout ou partie de ces zones peut ne pas être couvert par ce revêtement.

Bien que le revêtement s'étende sur une fraction substantielle de la longueur de la portion de travail, il est discontinu en raison de la présence de bandes annulaires 10 où ce revêtement n'est pas présent (ou quasiment pas présent). La surface de ces bandes est donc très légèrement en retrait par rapport à la surface extérieure des zones où le revêtement est présent, d'à peine l'épaisseur du revêtement.

Ces bandes sont ici disposées de manière régulière, à partir d'une certaine distance de l'extrémité (en effet la portion extrême de la portion de travail est entièrement revêtue).

Ces bandes ont ici une longueur (c'est-à-dire une dimension parallèle à la longueur de l'outil) au moins approximativement égale à leur écartement longitudinal, de sorte que chacun des bords de ces bandes constitue en soi un repère indicateur de profondeur ; ces bandes ont ici une longueur de 2 mm et un intervalle de 2 mm. En variante, la dimension longitudinale des bandes peut varier d'une bande à l'autre, en étant avantageusement comprise entre 1 mm et 3 mm (bornes comprises) ; de même la dimension longitudinale des intervalles peut varier d'un intervalle à l'autre, en étant avantageusement comprise entre 1 mm et 3 mm ; il s'agit de valeurs permettant une bonne distinction entre les bandes successives, tout en permettant une bonne détection de la bande à ne pas dépasser dans une application donnée.

Ces bandes 10 présentent un contraste avec les portions revêtues en raison de ce que le matériau constituant la portion de travail de l'outil présente un fort contraste avec le revêtement en CTEC.

Ces bandes peuvent être obtenues en ne déposant ce revêtement que sur l'extrémité étroite de la partie de travail et entre les bandes claires 10 ; toutefois, il est plus facile de procéder en déposant le revêtement sur la totalité des zones claires et foncées de la portion de travail, puis d'enlever sélectivement le revêtement à l'emplacement des bandes 10.

Le revêtement peut être déposé par toute technique connue appropriée, par exemple par dépôt en phase vapeur (ou CVD, acronyme de « Chemical Vapor Déposition »). Il s'agit d'un dépôt en couche mince, par exemple effectué sous un vide de l'ordre de 10⁻² bars à partir d'une cible en carbure de tungstène, en y adjoignant un gaz carboné ionisé qui confère au revêtement son caractère enrichi en carbone.

Ce revêtement a avantageusement une épaisseur comprise entre 1 et 5 micromètres, de préférence entre 1 et 3 micromètres, par exemple sensiblement égale à 3 micromètres.

L'enlèvement sélectif de ce revêtement, à l'emplacement des futures bandes claires 10, peut se faire par décapage chimique, mécanique ou par décapage laser. En particulier un décapage par laser permet d'enlever le revêtement non seulement sur la surface extérieure de la portion de travail mais aussi à l'intérieur des gorges longeant les arêtes de coupe.

De manière surprenante, il est apparu qu'un outil dentaire ainsi revêtu par du CTEC conduisait à des caractéristiques de frottement tout à fait comparables à celles d'un outil dentaire revêtu de carbone dur DLC, tout en présentant une meilleure résistance à l'usure et surtout en offrant un meilleur contraste visuel entre les bandes claires et les bandes foncées revêtues.

Dans leur démarche visant à mettre au point un outil dentaire présentant un meilleur contraste entre les bandes indicatrices de profondeur et le reste du revêtement, les inventeurs étaient initialement partis du postulat qu'il fallait un revêtement au moins aussi foncé que le DLC, alors que le DLC est connu pour avoir une couleur noir profond.

Les inventeurs ont donc cherché à identifier un autre revêtement ayant aussi une couleur bien noire ; cela les a amenés à s'intéresser à un alliage de type nitrure de titane-aluminium AlTiN, en testant plusieurs nuances disponibles sur le marché.

Il a été constaté que le décapage du revêtement aux emplacements des bandes à rendre claires était, selon les nuances testées, efficace ou non, ce qui a conduit à tester les nuances difficiles à décaper sans utiliser de couche d'accroche, ou avec une couche d'accroche au chrome.

Avec une couche d'accroche classique (TiN, d'épaisseur typiquement égale à 0,2 micromètres), il a été constaté qu'il subsistait, sur les zones décapées, une teinte jaune provenant de ce que le décapage laser brûlait cette couche d'accroche ; c'est pourquoi il a été tenté d'utiliser une couche d'accroche au chrome, faisant apparaître une teinte argentée plus proche de la teinte de l'outil dentaire nu. Le revêtement restait, dans son ensemble, biocompatible, mais cette teinte argent ne permettait pas d'avoir un meilleur contraste qu'avec du DLC. En l'absence de couche d'accroche, la tenue mécanique du revêtement était insuffisante.

Une des raisons auxquelles a été attribué le contraste insuffisant de cet alliage AlTiN a été que ce matériau est plus gris que noir.

Lorsqu'il a été envisagé de tester un revêtement en CTEC, il y a eu d'abord un a priori négatif en raison de ce que ce matériau est connu pour ne pas avoir des propriétés mécaniques aussi bonnes que du DLC ; on peut à cet égard se référer au document US - 5 299+ 937 qui, à propos de l'intérêt d'un revêtement de type DLC, prend l'exemple d'un outil en carbure de tungstène ; en outre, ce matériau présentait a priori le même inconvénient que le nitrure de titane-aluminium, à savoir celui d'avoir une teinte plus grise que noire. Enfin, le carbure de tungstène était connu pour contenir souvent du cobalt (à raison de son rôle de liant, à quelque pour-cents), ce qui le rendait a priori incompatible avec des applications impliquant une biocompatibilité. Enfin, la dureté du carbure de tungstène est inférieure à celle du DLC, surtout du DLC hydrogéné (1500HV contre 2200HV). Tout cela conduisait donc à conclure que ce revêtement était a priori impropre à l'application dans laquelle on cherchait à obtenir un meilleur contraste qu'avec du DLC.

Toutefois, lorsqu'il a été admis de conduire effectivement des tests, il a été constaté que les nuances actuelles de carbure de tungstène ne contiennent pas de cobalt surtout après dépôt sous vide (de type CVD, voir plus haut) et, après avoir adapté les conditions de décapage du revêtement en fonction de ce matériau, il est apparu que le contraste visuel entre les bandes décapées et les zones revêtues était meilleur, dans un grand nombre de cas, qu'avec du DLC ; en outre, le revêtement de CTEC est apparu avoir une plus grande résistance à l'usure que le DLC, ce qui semblait être en contradiction avec le fait qu'il était moins dur que ce matériau. Enfin ce matériau est apparu être biocompatible.

En fait, il semble que le revêtement en CTEC donne lieu très rarement à des reflets malgré la puissance de l'éclairage des scialyitiques ; ce revêtement a en effet un aspect mat. En outre, le décapage de ce revêtement ne conduit pas à un quelconque jaunissement de la surface décapée de sorte que ces surfaces sont plus claires que les zones décapées avec un matériau tel que AlTiN. A strictement parler, un tel revêtement en CTEC présente, par rapport à la surface sous-jacente (en pratique la surface d'un acier inoxydable), une différence de couleur inférieure à celle obtenue avec du DLC, mais l'impression d'un contraste meilleur provient de l'aspect mat du revêtement qui permet de conserver la détectabilité des bandes claires dans toutes les configurations d'éclairage, ce qui n'est pas le cas avec le DLC dont l'aspect est parfois brillant au point de présenter des reflets.

Il mérite d'être noté que, bien qu'il ait été proposé de former par décapage laser les bandes claires devant être formées sur un outil dentaire muni d'un revêtement en DLC (voir par exemple le document EP - 1 128 777), il est apparu qu'en pratique (probablement pour des raisons de difficulté technique ou de coût) de telles bandes claires sont obtenues au moyen d'un usinage mécanique, ce qui se reconnaît au fait que le revêtement reste intact dans les gorges longeant les arêtes de coupe (d'où une moindre visibilité des bandes claires) ; or un tel usinage, si l'on veut garantir un enlèvement total du revêtement pour obtenir un bon contraste de couleur, pénètre nécessairement dans la masse de l'outil, ne serait-ce que de quelques dizaines de microns, avec le risque d'enlever localement la couche de protection contre la corrosion et de former des amorces de rupture conduisant à des risques de casse de l'outil en service, ce qui peut se révéler rédhibitoire ; à cela s'ajoute que les marches générées par un tel usinage génère un risque de détachement du revêtement lorsque l'outil dentaire est en service. Or il est apparu que, avec un revêtement en CTEC, un décapage laser permet d'éliminer efficacement ce revêtement, y compris dans les gorges longeant les arêtes de coupe, sans dégrader le matériau du point de vue de la résistance à la corrosion ou de la résistance mécanique notamment en flexion. Il est à la portée de l'homme de métier de définir des conditions opératoires pour un tel décapage en fonction du matériau sous-jacent, en pratique un acier inoxydable.

Pourtant, le dépôt d'un revêtement en CTEC se fait à une température supérieure à celle du dépôt d'un DLC (de l'ordre de 400°C contre de l'ordre de 200°C) avec une technique impliquant généralement plus d'énergie que le dépôt de DLC (en dépôt en phase vapeur assisté par plasma, soit PACVD pour « Plasma Assisted Chemical Vapor Deposition »), ce qui laissait supposer que le dépôt de CTEC serait plus difficile à décaper que le revêtement de DLC. Pourtant il semble qu'il y ait, dans l'application envisagée, une synergie entre les inconvénients prévisibles du revêtement en CTEC, à savoir que la haute température de dépôt du revêtement en CTEC conduit à une meilleure accroche de ce revêtement sur la surface de l'outil ; il en découle que ce revêtement a une meilleure résistance à l'usure que le DLC ; en parallèle, le fait que le CTEC est moins dur que le DLC a pour conséquence qu'il se décape plus facilement que le DLC sans jaunissement ou ternissement de la surface sous-jacente, d'où la combinaison finale de propriétés qui contrairement à ce qu'on pouvait prévoir, est meilleure avec le CTEC qu'avec le DLC. Il peut être ajouté que le fait que l'usure du revêtement en CTEC soit plus lente que celle du DLC a pour avantage de préserver plus longtemps la qualité de coupe d'un outil muni du revêtement de l'invention par rapport à un outil revêtu de DLC.

Le CTEC a un coefficient de frottement de 0,15, à comparer avec la valeur de 0,1 pour le DLC, ce qui ne constitue pas une différence déterminante et le revêtement en CTEC offre une conductivité thermique comparable à celle du DLC.

Il peut être noté que le CTEC a une structure métallique (celle du carbure de tungstène) alors qu'un revêtement tel que celui préconisé dans l'art antérieur, c'est-à-dire un DLC, a une structure amorphe.

On comprend aisément que les commentaires ci-dessus s'appliquent non seulement à un foret tel que représenté mais aux autres types d'outils dentaires pouvant être utilisés en ostéotomie.

## Revendications

1. Instrument dentaire pour réaliser une ostéotomie comportant une tige de montage (2) et, dans le prolongement de celle-ci, une portion de travail (3) comportant des zones de découpe (3A), dont au moins une partie est recouverte par un revêtement en carbure de tungstène enrichi en carbone à l'exception d'une pluralité de bandes annulaires (10) réparties le long de la longueur de la portion de travail pour indiquer la profondeur de pénétration de l'outil dans une portion de mâchoire.

2. Instrument dentaire selon la revendication 1, dans lequel le revêtement a une épaisseur comprise entre 1 et 5 micromètres.

3. Instrument dentaire selon la revendication 1 ou la revendication 2, dans lequel les bandes annulaires sont équidistantes.

4. Instrument dentaire selon la revendication 3, dans lequel les bandes annulaires ont une dimension longitudinale égale à leur écartement.

5. Instrument dentaire selon l'une quelconque des revendications 1 à 4, dans lequel chaque écartement entre deux bandes annulaires successives est compris entre 1 et 3 mm.

6. Instrument dentaire selon l'une quelconque des revendications 1 à 5 dans lequel la dimension longitudinale de chaque bande est avantageusement comprise entre 1 et 3 mm.

## Patentansprüche

1. Zahnärztliches Instrument zur Durchführung einer Osteotomie, umfassend einen Befestigungsschaft (2) und in seiner Verlängerung einen Arbeitsabschnitt (3), der Schneidezonen (3A) umfasst, von denen zumindest ein Teil mit einer Beschichtung aus mit Kohlenstoff angereichertem Wolframcarbid bedeckt ist, mit Ausnahme einer Mehrzahl von ringförmigen Streifen (10), die entlang der Länge des Arbeitsabschnittes verteilt sind, um die Eindringtiefe des Werkzeugs in einen Abschnitt des Kiefers anzuzeigen.

2. Zahnmedizinisches Instrument nach Anspruch 1,
in welchem die Beschichtung eine Dicke zwischen 1 und 5 Mikrometer umfasst.

3. Zahnmedizinisches Instrument nach Anspruch 1 oder Anspruch 2,
in welchem die ringförmigen Streifen gleich beabstandet sind.

4. Zahnmedizinisches Instrument nach Anspruch 3,
in welchem die ringförmigen Bänder eine Längsabmessung aufweisen, die gleich ihrem Abstand ist.

5. Zahnmedizinisches Instrument nach einem der Ansprüche 1 bis 4,
in welchem jeder Abstand zwischen zwei aufeinander folgenden ringförmigen Streifen zwischen 1 und 3 mm umfasst.

6. Zahnmedizinisches Instrument nach einem der Ansprüche 1 bis 5,
in welchem die Längsabmessung jedes Streifens vorteilhaft zwischen 1 und 3 mm umfasst.

## Claims

1. A dental instrument adapted for performing osteotomy comprising a mounting shank (2) and, in line therewith, a working portion (3) comprising cutting zones (3A), of which at least part is covered by a coating of carbon-enriched tungsten carbide except for a plurality of annular bands (10) distributed along the length of the working portion to indicate the depth of penetration of the tool into a portion of jaw.

2. A dental instrument according to claim 1, wherein the coating is of a thickness comprised between 1 and 5 micrometers.

3. A dental instrument according to claim 1 or claim 2, wherein the annular bands are equidistant.

4. A dental instrument according to claim 3, wherein the annular bands have a longitudinal dimension equal to their separation.

5. A dental instrument according to any one of claims 1 to 4, wherein, each separation between two successive annular bands is comprised between 1 and 3 mm.

6. A dental instrument according to any one of claims 1 to 5, in which the longitudinal dimension of each band is advantageously comprised between 1 and 3 mm.
